# EUROPEAN PATENT APPLICATION

(11) **EP 1 719 513 A1**
(43) Date of publication of application: **08.11.2006**
(21) Application number: 05710610.6
(22) Date of filing: 17.02.2005
(51) Int. Cl.: A61K 31/593, A61P 3/02, A61P 3/14

(54) **METHOD OF PREVENTING, MEDICATING AND/OR TREATING HYPOCALCAEMIA OF DOMESTIC MAMMAL**

(30) Priority: 18.02.2004 JP 2004041202
(71) Applicant: MERCIAN CORPORATION, Chuo-ku, Tokyo 104-8305 (JP)
(72) Inventor: NAITO, Yoshihisa, Ofunato-shi, Iwate 022-0101 (JP); YAMAGISHI, Norio, Obihiro-shi, Hokkaido 080-0026 (JP); OKURA, Norimoto, Asahikawa-shi, Hokkaido 078-8822 (JP)
(74) Representative: Horner, Martin Grenville
(86) International application number: PCT/JP2005/002959
(87) International publication number: WO 2005/077378

(57) **Abstract**

The present invention provides a method of preventing, curing, and/or treating hypocalcemia of a domestic mammal, especially, a cow.

Specifically, the present invention provides a method of administrating a vitamin D derivative to prevent, cure and/or treat hypocalcemia, **characterized in that** a vitamin D derivative, especially, 1α-hydroxyvitamin D₃ and/or 1.25-dihydroxyvitamin D₃ is transvaginally administered to a domestic mammal.

## Description

### Technical Field

The present invention relates to a method of preventing, curing, and/or treating hypocalcemia of a domestic animal. More specifically, the present invention relates to a method of preventing, curing, and/or treating hypocalcemia of a domestic mammal by transvaginally administering a vitamin D derivative to the domestic mammal.

### Background Art

In general, administration of vitamin D₃ (hereinafter, also abbreviated to VD₃) is used as one of methods of preventing, curing, and/or treating parturient hypocalcemia of dairy cows.

VD₃ is metabolized to 25-hydroxyvitamin D₃ in a liver and further to 1,25-dihydroxyvitamin D₃ (hereinafter, also abbreviated to 1,25-(OH)₂D₃) in a kidney.

A method of preventing, curing, and/or treating hypocalcemia by administering 1,25-(OH)₂D₃, the most physiologically active metabolite among VD₃ metabolites that regulate calcium metabolism, to a daily cow is also practiced (see JP-A 61-233620 (related application: US Patent No. 322462) and Gast et al., J. Dairy Sci. vol. 62: pp. 1009-1013, 1979).

The administration of a drug via the vagina has been known since ancient Egypt. During the last century, the absorption of many substances such as estrogen, progesterone, prostaglandin, antibiotics, nonoxynol-9, methasone, and inorganic compounds from the vagina had been observed in humans and animals.

In recent years, an intravaginal insert containing progesterone is widely used for regulating the estrous cycle of a cow (see JP 2001-523515 T (related application: International Publication of WO99/26556) and J. Dairy Sci. vol. 70: pp. 2162-2167, 1987).

Although 1,25-(OH)₂D₃ has been used in intravenous, intramuscular, and oral administration methods for preventing, curing, and/or treating parturient hypocalcemia of dairy cows, the effectiveness of intravaginal administration of 1,25-(OH)₂D₃ against parturient hypocalcemia has not been found.

### Disclosure of the Invention

An object of the present invention is to provide a method of readily preventing, curing, and/or treating hypocalcemia of a domestic mammal, especially diseases such as intrapartum astasia that is a leading cause of death and disuse of cows, by transvaginally administering a vitamin D derivative to the domestic mammal.

The inventors of the present invention have intensively studied the absorptivity of vitamins D, A, and E, which are fat-soluble vitamins, by transvaginally administering them to cows. As a result, the inventors of the present invention have completed the present invention by finding that the absorptivity of those fat-soluble vitamins is generally low and however, only a particular vitamin D derivative is favorably absorbed from the vagina with ease and is effective for preventing, curing, and/ or treating diseases associated with hypocalcemia.

That is, the present invention provides a method of administering a vitamin D derivative according to any one of items 1 to 15 below, which is useful for preventing, curing, and/or treating diseases associated with hypocalcemia (especially astasia or the like of a cow) of a domestic mammal, for example, a cow, a horse, a sheep, a goat, a pig, a dog, and a cat, by transvaginally administering the vitamin D derivative to the domestic mammal.
1. A method of preventing hypocalcemia, characterized in that a vitamin D derivative is transvaginally administered to a domestic mammal.
2. The method of preventing hypocalcemia according to the above item 1, in which the domestic mammal is a cow.
3. The method of preventing hypocalcemia according to the above item 1 or 2, including administering an intravaginal insert containing the vitamin D derivative to a vaginal cavity.
4. The method of preventing hypocalcemia according to any one of the above items 1 to 3, in which the vitamin D derivative administered intravaginally is absorbed in the vaginal cavity of the domestic mammal to increase a calcium concentration in the body, thereby preventing the disease.
5. The method of preventing hypocalcemia according to any one of the above items 1 to 4, in which the vitamin D derivative is 1α-hydroxyvitamin D₃ or 1,25-dihydroxyvitamin D₃.
6. A method of curing hypocalcemia, including transvaginally administering a vitamin D derivative to a domestic mammal.
7. The method of curing hypocalcemia according to the above item 6, in which the domestic mammal is a cow.
8. The method of curing hypocalcemia according to the above items 6 or 7, including administering an intravaginal insert containing the vitamin D derivative to a vaginal cavity.
9. The method of curing hypocalcemia according to any one of the above items 6 to 8, in which the vitamin D derivative administered intravaginally is absorbed in the vaginal cavity of the domestic mammal to increase a calcium concentration in the body, thereby curing the disease.
10. The method of curing hypocalcemia according to any one of the above items 6 to 9, in which the vitamin D derivative is 1α-hydroxyvitamin D₃ or 1,25-dihydroxyvitamin D₃.
11. A method of treating hypocalcemia, characterized in that a vitamin D derivative is transvaginally administered to a domestic mammal.
12. The method of treating hypocalcemia according to the above item 11, in which the domestic mammal is a cow.
13. The method of treating hypocalcemia according to the above item 11 or 12, including administering an intravaginal insert containing the vitamin D derivative to a vaginal cavity.
14. The method of treating hypocalcemia according to any one of the above items 11 to 13, in which the vitamin D derivative administered intravaginally is absorbed in the vaginal cavity of the domestic mammal to increase a calcium concentration in the body, thereby treating the disease.
15. The method of treating hypocalcemia according to any one of the above items 11 to 14, in which the vitamin D derivative is 1α-hydroxyvitamin D₃ or 1,25-dihydroxyvitamin D₃.

### Detailed Description of the Invention

A vitamin D derivative to be administered to the vaginal cavity of a domestic mammal in the present invention includes a vitamin D derivative represented by the following general formula (1): 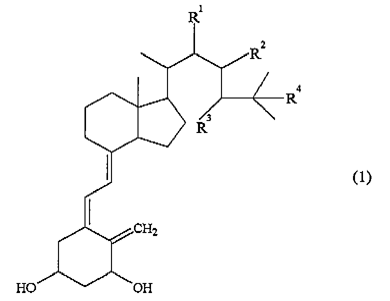 wherein R¹ and R² each represent a hydrogen atom or R¹ and R² together may form a double bond; R³ represents a hydrogen atom or a methyl group; and R⁴ represents a hydrogen atom or a hydroxyl group.

A concrete example of the vitamin D derivative represented by the above general formula (1) includes a calciferol derivative (vitamin D₂ derivative) wherein R¹ and R² together form a double bond and R³ represents a methyl group or a cholecalciferol derivative (vitamin D₃ derivative) wherein R¹, R², and R³ each represent a hydrogen atom.

Of those vitamin D₂ and D₃ derivatives, 1α-hydroxyvitamin D and/or 1,25-dihydroxyvitamin D derivatives are preferred. A typical example thereof includes 1α-hydroxyvitamin D₂, 1α-hydroxyvitamin D₃, or 1α,25-dihydroxyvitamin D₃.

The vitamin D derivative is administered to a vaginal cavity using an intravaginal insert containing the vitamin D derivative. The form of the intravaginal insert (delivery type) that can be used is, for example, a gel, a tablet, a microsphere, and CIDR, which are generally used.

The absorption of the vitamin D derivative from the vaginal mucous membrane can be confirmed by observing changes in the vitamin D derivative administered to the vaginal cavity and several types of minerals (calcium (Ca), inorganic phosphorus (iP), and magnesium (Mg)).

For example, the absorption can be confirmed by intravaginally administering 1,25-(OH)₂D₃ dissolved in ethanol at a dose of approximately 1 µg per kg of body weight to the vaginal cavity of a cow and comparing the cow with a control to which ethanol is administered.

In the cow to which 1,25-(OH) ₂D₃ is administered, the value of 1,25-(OH)₂D₃ in plasma changes and the values of Ca, iP, and Mg in plasma change. The absorption of 1,25-(OH)₂D₃ can be confirmed by observing the changes in the value of 1,25-(OH)₂D₃ in plasma.

### Brief Description of the Drawings

Fig. 1 shows the shift with time of a 1, 25-dihydroxyvitamin D₃ (1,25-(OH)₂D₃) concentration in blood for each test cow (Cow A or Cow B) by the intravaginal administration of 1α-VD₃ in Example 1.
Fig. 2 shows the shift with time of a calcium (Ca) concentration in blood for each test cow (Cow A or Cow B) by the intravaginal administration of 1α-VD₃ in Example 1.
Fig. 3 shows the shift with time of an inorganic phosphorus (iP) concentration in blood for each test cow (Cow A or Cow B) by the intravaginal administration of 1α-VD₃ in Example 1.
Fig. 4 shows the shift with time of a magnesium (Mg) concentration in blood for each test cow (Cow A or Cow B) by the intravaginal administration of 1α-VD₃ in Example 1.
Fig. 5 shows the shift with time of a vitamin A (VitA) concentration in blood for each test cow (Cow A or Cow B) by the intravaginal administration of VitAD₃E in Comparative Example 1.
Fig. 6 shows the shift with time of a vitamin E (VitE) concentration in blood for each test cow (Cow A or Cow B) by the intravaginal administration of VitAD₃E in Comparative Example 1.
Fig. 7 shows the shift with time of a 25-hydroxyvitamin D₃ (25-OHD₃) concentration in blood for each test cow (Cow A or Cow B) by the intravaginal administration of VitAD₃E in Comparative Example 1.
Fig. 8 shows the shift with time of a 1,25-(OH)₂D₃ concentration in plasma for raised cows to which 1,25-(OH)₂D₃ (n=5; ●) and ethanol (n=1; ■) are intravaginally administered in Example 2.
Fig. 9 shows the shift with time of mineral (Ca, iP, and Mg) concentrations in plasma for raised cows to which 1,25-(OH)₂D₃ (n=5; ●) and ethanol (n=1; ■) are intravaginally administered in Example 2.
Fig. 10 shows the shift with time of a 1,25-(OH)₂D₃ concentration in plasma for raised cows to which 1,25-(OH)₂D₃ is intravaginally administered in Example 3. However, in Fig. 10, "iv" denotes intravenous administration (ditto with Fig. 11 to Fig. 16).
Fig. 11 shows the shift with time of a calcium concentration in plasma for raised cows to which 1,25-(OH)₂D₃ is intravaginally administered in Example 3.
Fig. 12 shows the shift with time of an iP concentration in plasma for raised cows to which 1,25-(OH)₂D₃ is intravaginally administered in Example 3.
Fig. 13 shows the shift with time of a Mg concentration in plasma for raised cows to which 1, 25-(OH)₂D₃ is intravaginally administered in Example 3.
Fig. 14 shows the shift with time of a Ca/creatinine (Cre) concentration in urine for raised cows to which 1,25-(OH)₂D₃ is intravaginally administered in Example 3.
Fig. 15 shows the shift with time of an iP/creatinine (Cre) concentration in urine for raised cows to which 1,25-(OH)₂D₃ is intravaginally administered in Example 3.
Fig. 16 shows the shift with time of a Mg/creatinine (Cre) concentration in urine for raised cows to which 1,25-(OH)₂D₃ is intravaginally administered in Example 3.

### Best Mode for carrying out the Invention

Hereinafter, the present invention will further be described with reference to Examples and Comparative Example. However, the present invention is not intended to be limited to them by any means. In the examples described below, a 1,25-(OH)₂D₃ concentration in plasma was determined using a radioimmunoassay kit (1,25-(OH)₂D RIA kit, Immunodiagnostic Systems Limited, UK). The Ca concentrations, inorganic phosphorus (iP) concentrations, and magnesium (Mg) concentrations in plasma and urine were determined by orthocresolphthalein complexone (O-CPC), molybdenum (Mo), and xylidyl blue methods, respectively. A creatinine concentration in urine was determined by a Jaffé method. Calcium (Ca), inorganic phosphorus (iP), and magnesium (Mg) concentrations in urine were indicated in the ratios relative to creatinine (Cre) (Ca/Cre, iP/Cre, and Mg/Cre, respectively).

### Example 1

Biochemical changes in blood were observed by administering 1α-hydroxyvitamin D₃ to the vaginal cavities of cows.

Test animals used were two Holstein dairy cows (Cow A and CowB) describedbelow. During a test period, the cows were raised by the free grazing of hay (free water drinking) in a field paddock as well as feeding with 2 kg/day of formula feed (64.1% TDN, 13.6% CP, 0.2% Ca, 0.09% Mg, and 1.39% K in DM).

CowA: 35 months old, female, single birth, ovariectomized, 560 kg

CowB: 34 months old, female, singlebirth, ovariectomized, 580 kg

The following test drug was used: 1α-hydroxyvitamin D₃ (1α-VD₃; manufactured by Nisshin Pharma Co., Ltd., powder).

Test contents and schedules were as follows:
1. Control test: intravaginal administration of 4 mL of 20% ethanol (for 1 week); and
2. 1α-VD₃ test: intravaginal administration of 1µg/kg (by body weight) of 1α-VD₃ dissolved in 4 mL of 20% ethanol (for 2 weeks) .

Intravaginal administration was performed by the following procedure:
The above-described reagent was prepared on ice water and then administered, with a 10-mL plastic syringe, to a deep region in the vagina of the test cows previously allowed to urinate and vulva skin was immediately closed with an instant adhesive.
In each test, blood was collected immediately before administration (0 hour) and at 0.5, 1, 2, 3, 6, 12, 24, 48, and 72 hours (hrs) after administration.

The biochemical examination of blood was conducted on the following items:
Control test: 1,25-dihydroxyvitamin D₃ [1,25-(OH)₂D₃], calcium (Ca), inorganic phosphorus (iP), and magnesium (Mg); and 1α-VD₃ test: 1,25-(OH)₂D₃, Ca, iP, and Mg.

The obtained data was analyzed by comparing the result of the biochemical examination of blood for each test cow in the 1α-VD₃ test with the result of control test and investigating the presence or absence of the intravaginal absorption of each drug.

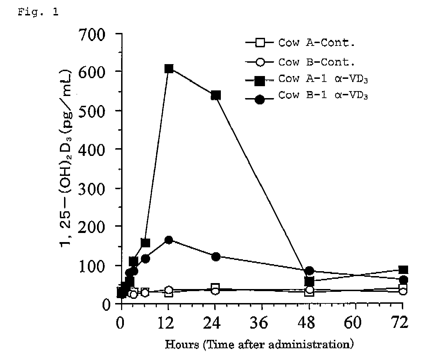

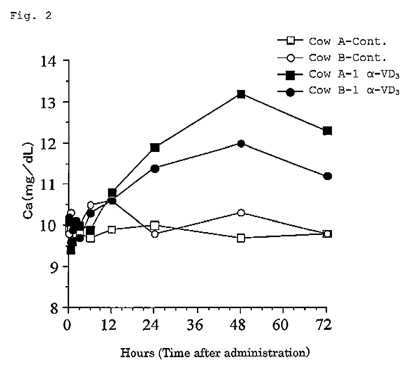

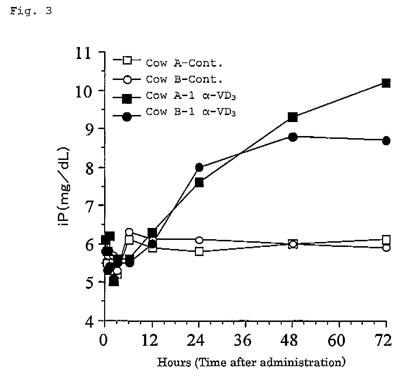

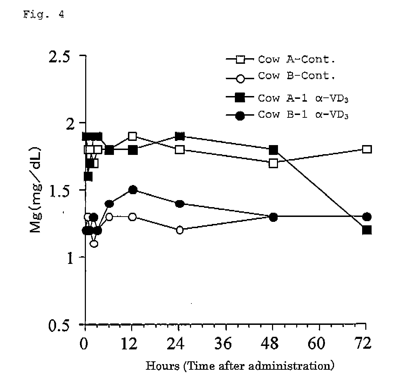

The shifts of 1,25-(OH)₂D₃, Ca, iP, and Mg concentrations in blood by the intravaginal administration of 1α-VD₃ were observed by the above-described procedure. Results obtained by observing the shifts of concentrations by the intravaginal administration of 1α-VD₃ are shown in Fig. 1 to Fig. 4.

Comparison and contemplation between the result of the present Example 1 and the result of Comparative Example 1 below demonstrate that 1α-VD₃ is absorbed from the vaginal wall into the body by the intravaginal administration of 1α-VD₃ and quickly converted to 1,25-(OH)₂D₃, thereby affecting Ca metabolism.

No evidence that administered drugs were absorbed from the vaginal wall was confirmed in the intravaginal administration of vitamin A, vitamin D₃, and vitamin E in Comparative Example 1 below.

### Comparative Example 1

Vitamin A, vitamin D₃, and vitamin E were administered to the vaginal cavities of cows to observe biochemical changes in blood after administration in the same manner as in Example 1 except that test drugs, test contents, and biochemical examination items of blood were as described in the following (1) to (3).
(1) Test drug
   Vitamin A (VitA; bulk for drug production, liquid);
   Vitamin D₃ (VitD₃; bulk for drug production, liquid); and
   Vitamin E (VitE; bulk for drug production, liquid).
(2) Test contents
   VitAD₃E test: intravaginal administration of VitA (10,000,000 IU)+VitD₃ (5,000,000 IU)+VitE (920 IU) brought up to 8 mL per cow with 20% ethanol.
(3) Biochemical examination items of blood
   Control test: VitA, 25-hydroxyvitamin D₃ (25-OHD₃), VitE, calcium (Ca), inorganic phosphorus (iP), and magnesium (Mg); and VitAD₃E test: VitA, 25-OHD₃, and VitE.

The obtained data was analyzed by comparing the result of the biochemical examination of blood for each test cow in the VitAD₃E test with the result of control test and investigating the presence or absence of the intravaginal absorption of each drug.

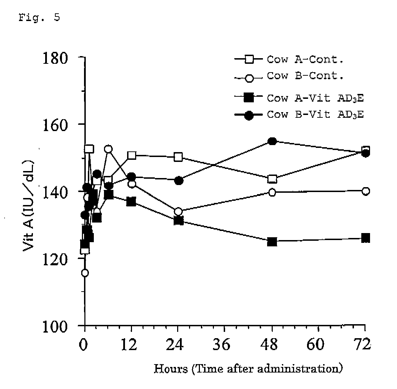

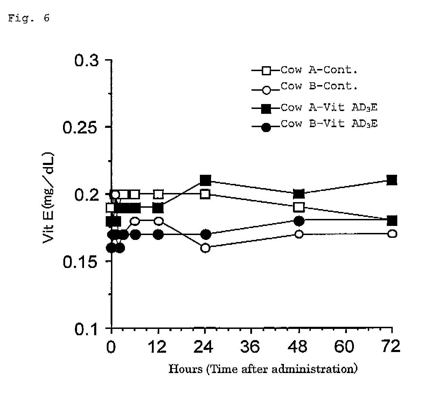

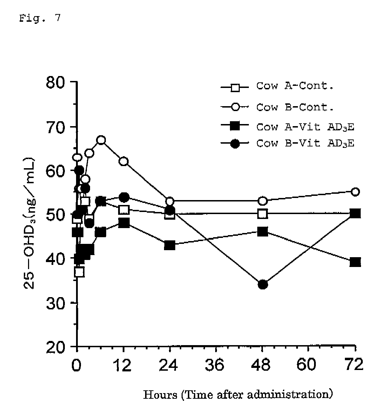

The shifts of VitA, 25-OHD₃, and VitE concentrations in blood by the intravaginal administration of VitAD₃E were observed by the above-described procedure. Results obtained by observing the shifts of concentrations by the intravaginal administration of VitAD₃E are shown in Fig. 5 to Fig. 7.

Comparison and contemplation between the result of the present Comparative Example 1 and the result of the above Example 1 demonstrate that 1α-VD₃ in Example 1 is absorbed from the vaginal wall into the body by the intravaginal administration of 1α-VD₃ and quickly converted to 1,25-(OH)₂D₃, thereby affecting Ca metabolism, whereas no evidence that the administered drugs were absorbed from the vaginal wall was confirmed in the intravaginal administration of VitAD₃E in Comparative Example 1.

### Example 2

For confirming the absorption in the vagina of 1,25-(OH)₂D₃ administered to the vaginal cavities of cows, biochemical changes of 1,25-(OH)₂D₃ and minerals in blood were observed after the intravaginal administration of 1,25-(OH)₂D₃ to cows.

Six Holstein cows (3 to 6 months olds, 97 to 118 kg in body weights) that were clinically healthy were raised and domesticated for at least 1 week in the same fence by providing them with feed (1.56 kg of grass, 0.55 kg of formula feed, and 1.44 kg of alfalfa hey cube in dried forms) having a daily mineral intake of 21 g of Ca, 13 g of P, and 4 g of Mg that satisfies requirements stipulated by NRC (National Research Council) on a daily basis and free water drinking.

To each of five of the cows, 1,25-(OH)₂D₃ (crystal manufactured by Mercian Corporation dissolved in 99% ethanol to bring its concentration to 1 mg/mL and cryopreserved at -20°C until just before use) was intravaginally administered at 1 µg per kg of body weight.

Intravaginal administration was performed using a 14-gauge, 64 mm-long cannula with an indwelling needle for injection (Surflo, Terumo Co. Ltd., Tokyo) and a plastic pump (Top Plastic Syringe, Top Surgical Taiwan Corporation, Taiwan).

To the other cow, 3.0 mL of 99% ethanol was administered as a control.

A heparinized blood sample was collected from a jugular vein immediately before administration (0 hour) and at 2, 6, 12, 24, 48, 72, and 96 hours after administration.

The biochemical value of blood was indicated as a means ± standard deviation. For observing the effect of the intravaginal administration of 1,25-(OH)₂D₃, repeated measures analysis of variance was used. When the effect was significant, a statistical test was conducted by the Dunnett's multiple comparison between the value at 0 hour and each value after administration. The significant difference was set to P<0.05.

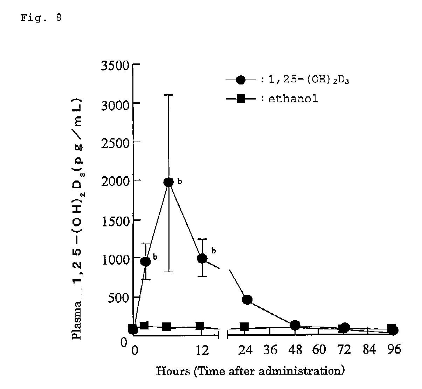

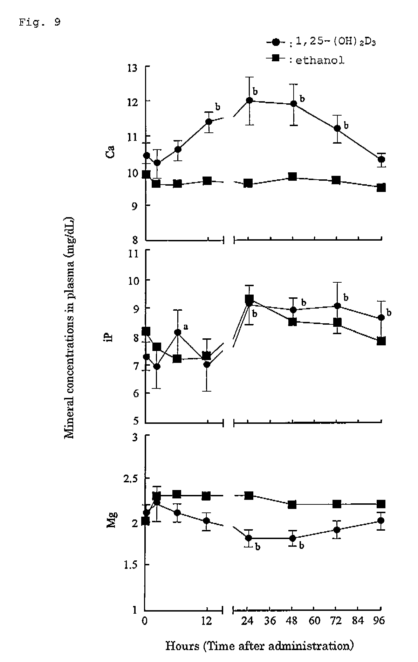

Although significant changes in 1,25-(OH)₂D₃, Ca, iP, and Mg concentrations in plasma were observed in the cows to which 1,25-(OH)₂D₃ was intravaginally administered, those varying concentrations in plasma were not affected by ethanol administration except for iP (Fig. 8 and Fig. 9).

As can be seen from Fig. 8, a significant change (b) P<0.01) was observed in the value of the 1,25-(OH)₂D₃ concentration in plasma of the cows to which 1,25-(OH)₂D₃ was intravaginally administered, as compared with the value at 0 hour.

As can be seen from Fig. 9, significant changes (a) P<0.05 and b) P<0.01) were observed in the values of the mineral (Ca, iP, and Mg) concentrations in plasma of the cows to which 1,25-(OH)₂D₃ was intravaginally administered, as compared with the value at 0 hour.

The value of 1,25-(OH)₂D₃ in plasma was 88.3±20.3 pg/mL before administration (0 hour) and significantly (p<0.01) increased to 1967.4±1139.6 pg/mL at 6 hours after administration of 1,25-(OH)₂D₃, and reduced thereafter.

The Ca concentration in plasma of the cows to which 1,25-(OH)₂D₃ was administered was significantly (P<0.01) high at 12 to 72 hours after administration as compared with the value before administration (10.4±0.4 mg/dL) and exhibited the maximum value (11.96±0.7 mg/dL) at 24 hours after administration.

Change in the iP concentration in plasma observed in the cow to which 1,25-(OH)₂D₃ was administered was same as that of the cow which underwent ethanol administration.

The value of iP in plasma was significantly high at 6 hours (8.1±0.8 mg/dL; P<0.05) and 24 to 96 hours (9.1±0.7 to 8.6±0.6 mg/dL; P<0.01) after administration of 1,25-(OH)₂D₃ as compared with the value at 0 hour (7.3±0.5 mg/dL).

The value of Mg in plasma was significantly (P<0.01) low at 24 and 48 hours (1.8±0.1 and 1.8±0.1 mg/dL) after administration of 1,25-(OH)₂D₃ as compared with the value at 0 hour (2.1±0.1 mg/dL).

In the present result, a sudden increase and rise of 1,25-(OH)₂D₃ in plasma were observed only in the cows at 2 hours after administration of 1,25-(OH)₂D₃.

With that, the absorption of 1,25-(OH)₂D₃ from the vaginal wall of a cow has been confirmed.

It is noted that the state of the above-described changes in the 1,25-(OH)₂D_{B} concentration in plasma is similar to results observed in nonpregnant-nonlactating adult cows intramuscularly injected with 1,25-(OH)₂D₃. The main physiological effect of 1,25-(OH)₂D₃ is to increase Ca and iP concentrations in plasma bybeingabsorbedfrom theintestinaltract. However, the present result from the raised cows to which 1,25-(OH)₂D₃ was intravaginally administered was similar to the values of Ca and iP in plasma that were led to high levels by the intravenous injection of 1,25-(OH)₂D₃.

However, in the present experimental result, an initial decrease and subsequent increase in an iP concentration in plasma were observed in the raised cow to which ethanol was administered.

In similar biphasic changes in the value of iP in plasma obtained with rabbits, it is considered that hypophosphatemia was caused at an early stage by the metabolic process of ethanol catalyzed by ethanol dehydrogenase and hyperphosphatemia was subsequently induced by acetaldehyde, an ethanol metabolite thereof.

Therefore, the change in the iP concentration in plasma in the present experiment suggests that not only 1,25-(OH)₂D₃ but ethanol was absorbed via the vaginal wall of a cow.

The cause of hypomagnesemia after the intramuscular injection or intravaginal administration of 1,25-(OH)₂D₃ to an adult cow is not elucidated. However, hypomagnesemia may be due to 1,25-(OH)₂D₃ that decreases the reabsorption of Mg in the renal tubule and thereby increases the renal excretion of Mg.

The thickness of the vaginal epithelium of a cow is considered to vary in response to the secretion of ovarian hormones. The raised cows used in the present Example do not reach puberty. Therefore, the absorption of 1,25-(OH) ₂D₃ from the vagina results in no change in the thickness of the vaginal epithelium. Thus, the absorption is considered to be more stable in the raised cows having the thin vaginal epithelium than those in adult cows. However, the present experimental result indicates that the intravaginal administration of 1,25-(OH)₂D₃ may be sufficient for preventing parturient hypocalcemia.

### Example 3

The dose-response test of 1,25-(OH)₂D₃ by an intravaginal administration route was performed with five 3 to 9-year-old ovariectomized Holstein cows having body weights of 616 to 804 kg as test animals.

The cows were hitched to a partition after and provided with 5.3 kg of timothy hay, 0.18 kg of alfalfa hay, 0.71 kg of beet pulp pellet, and 1.7 kg of commercially-available grain mix on a daily basis. The cows were measured on a DM basis and freely provided with water. A daily mineral intake was set to 48.4 g for calcium, 20.2 g of inorganic phosphorus, and 12.7 g for magnesium, which sufficiently exceeded the NRC recommendation.

To the five cows, 1,25-(OH)₂D₃ was respectively administered at 0.125, 0.25, 0.5, and 1.0 µg/kg (by body weight) as an intravaginal dose level and 1.0 µg/kg (by body weight) as an intravenous injection dose level at an interval of 2 weeks or more according to a 5x5 Latin square design.

The 1,25-(OH)₂D₃ (manufactured by Mercian Corporation) used was in the form of crystalline powder, which was dissolved in 99% ethanol at 200 µg/mL and cryopreserved at -20°C until use.

A drug composed of 5 mL of 20% ethanol solution containing 1,25-(OH)₂D₃ at 0.125, 0.25, 0.5, or 1.0 µg/kg (by body weight) was administered to a vaginal lumen using a Split Universal Sheath (IMV Int. CO., France) by a rectovaginal cavity method. The vulva was then bonded with an adhesive in order to prevent the 1,25-(OH)₂D₃ solution from being unintentionally excreted from the vaginal lumen. Intravenous administration was performed using a cannula (14-ga cannula for animals, manufactured by Nipro Medical Industries Ltd.) mounted in advance for the collection of a blood sample.

A heparinized blood sample was collected through the cannula immediately before the administration of 1,25-(OH)₂D₃ (0 hour) and at 2, 4, 6, 12, 24, 48, 72, 96, and 120 hours after administration.

Next, the blood was immediately centrifuged, and 1,25-(OH)₂D₃, calcium, inorganic phosphorus, and magnesium concentrations in plasma were determined.

Urine were collected by urethral catheterization simultaneously with the collection of blood samples from the cows that underwent the intravaginal administration of 1,25-(OH)₂D₃ at 0.125 and 1.0 µg/kg (by body weight) and the intravenous injection of 1,25-(OH)₂D₃ at 1. 0 µg/kg (by body weight), to determine creatinine, calcium, inorganic phosphorus, and magnesium concentrations in the urine. Plasma and urine samples were cryopreserved at -20°C until analysis.

As a result, there was the significant difference of changes in 1,25-(OH)₂D₃, calcium, inorganic phosphorus, and magnesium concentrations in plasma of the cows to which four levels of 1,25-(OH)₂D₃ were intravaginally administered. However, a difference among groups to which different levels of 1,25-(OH) ₂D₃ were administered was not significant in plasma except for 1,25-(OH)₂D₃. Similarly, although there was significant difference of changes in calcium, inorganic phosphorus, and magnesium in plasma of the cow to which 1,25-(OH)₂D₃ was intravenously administered, no difference arose between groups of intravaginal administration and intravenous administration.

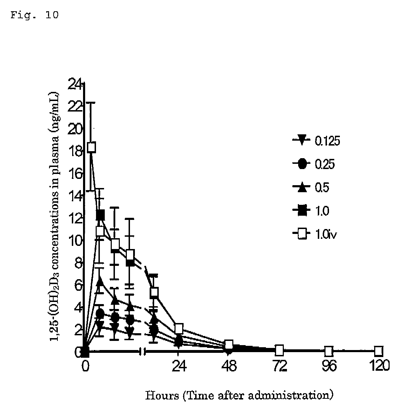

When 1,25-(OH)₂D₃ was intravaginally administered at 0.125, 0.25, 0.5, and 1.0 ug/kg (by body weight), 1,25-(OH)₂D₃ levels in plasma significantly increased from 2 hours to 24 hours after treatment as compared with 0 hour (7.4±5.3, 6.5±1.3, 8.7±5.6, and 6.6±1.6 pg/mL). Those levels reached peaks (2219.3±812.0, 3448.7±737.9, 6388.5±1127.4, and 12315.7±2288.3pg/mL) at 2 hours after administration, and reduced thereafter. There was a significant difference among groups to which 1,25-(OH)₂D₃ were administered at 0.125x0.5, 0.125x1.0, 0.25x0.5, 0.25x1.0, and 0.5x1.0 µg/kg (by body weight) (Fig. 10). In the cow that underwent intravenous administration, 1,25-(OH)₂D₃ in plasma became similar to those intravaginally administered by 2 hours after administration and was then changed in a similar manner.

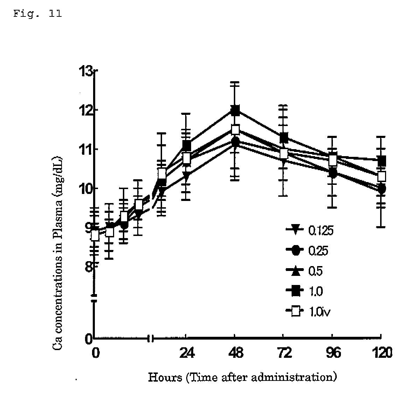

In intravaginal administration, a calcium concentration in plasma of the cow to which 1,25-(OH)₂D₃ was administered at 0.125 or 0.25 µg/kg (by body weight) was significantly high from 12 hours to 120 hours after administration as compared with 0 hour (8.9±0.5 or 8.9±0.4 mg/dL) and reached a peak (11.1±0.9 or 11.2±0.7 mg/dL) at 48 hours after administration. Alternatively, a calcium concentration in plasma of the cow to which 1,25-(OH)₂D₃ was administered at 0.5 or 1. 0 µg/kg (by body weight) was significantly high from 6 hours to 120 hours after administration as compared with 0 hour (8.9±0.2 or 8.8±0.7 mg/dL) and reached a peak (11.5±0.6 or 12.0±0.6 mg/dL) at 48 hours after administration. Changes in a calcium concentration (8.8±0.5 mg/dL at 0 hour, 11.5±1.2 mg/dL at a peak) in plasma of the cow to which 1,25-(OH)₂D₃ was intravenously administered were similar to those in the cows to which 1,25-(OH)₂D₃ was intravaginally administered at 0.5 or 1. 0 µg/kg (by body weight) (Fig. 11).

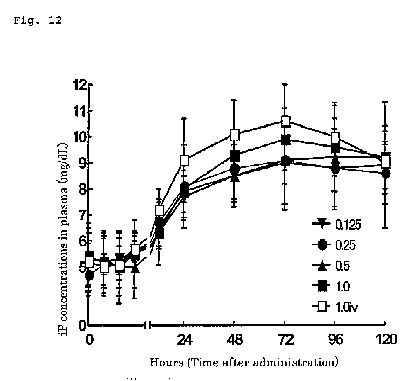

An inorganic phosphorus concentration (5.3±1.0, 5.3±0.6, or 5.4±1.3mg/dL at 0 hour) inplasmaof the cowtowhich 1,25-(OH)₂D₃ was administered at 0.125, 0.5, or 1.0 µg/kg significantly rose at 24 hours after intravaginal administration of 1,25-(OH)₂D₃ (7.7±0.8, 7.9±0.9, and 8.0±1.2mg/dL, respectively) and reached the maximum value from 24 hours to 120 hours after administration. An inorganic phosphorus level in plasma of the cow to which 1,25-(OH)₂D₃ was intravaginally administered at 0.25 µg/kg was significantly high (6.7±0.9 to 8.6±1.2 mg/dL) at 12 hours to 120 hours after administration as compared with the level at 0 hour (4.7±0.6 mg/dL). Changes in an inorganic phosphorus concentration (5.2±1.3 mg/dL at 0 hour) in plasma of the cow to which 1,25-(OH)₂D₃ was intravenously administered increased (7.2±0.8 mg/dL) at 12 hour after administration and reached a peak from 24 hours to 12 hours after administration (9.1±1.6 to 9.0±1.2 mg/dL) (Fig. 12).

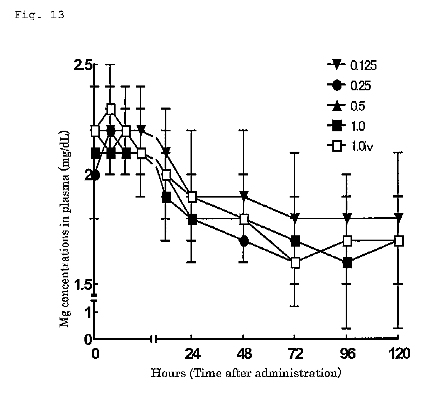

In intravaginal administration, a magnesium concentration in plasma of the cow to which 1,25-(OH)₂D₃ was administered at 0.125, 0.25, or 1.0 µg/kg was significantly low (1.9±0.1, 1.8±0.1, or 1.8±0.2 to 1.8±0.3, 1.7±0.2, or 1.7±0.4 mg/dL) from 24 hours to 120 hours after administration as compared with the respective values at 0 hour (2.2±0.2, 2.0±0.2, and 2.1±0.1 mg/dL). A magnesium concentration in plasma of the cow to which 1,25-(OH) ₂D₃ was administered at 0.5 µg/kg was significantly low (1.9±0.2 to 1.7±0.2 mg/dL) from 12 hours to 120 hours after administration as compared with the value at 0 hour (2.2±0.2 mg/dL). Changes in a magnesium concentration (2.2±0.2 mg/dL at 0 hour, 1.9±0.3 to 1.7±0.2 mg/dL from 24 hours to 120 hours after administration) in plasma of the cow to which 1,25-(OH)₂D₃ was intravenously administeredwere similar to those in the cow to which 1,25-(OH)₂D₃ was intravaginally administered at 0.125, 0.5 or 1.0 µg/kg by body weight (Fig. 13).

Significant changes were observed in the values of a calcium/ creatinine ratio (Ca/Cre), an inorganic phosphorus/creatinine ratio (iP/Cre), and a magnesium/creatinine ratio (Mg/Cre) in urine in both of the cow to which 1,25-(OH)₂D₃ was intravaginally administered at 1.0 µg/kg by body weight and the cow to which 1,25-(OH)₂D₃ was intravenously administered at 1. 0 µg/kg by body weight. There was no influence on those values in the cow to which 1,25-(OH)₂D₃ was intravaginally administered at 0.125 µg/kg by body weight. There was a significant difference on the value of Mg/Cre among groups in the cow to which 1,25-(OH)₂D₃ was intravaginally administered at 0.125 or 1.0 µg/kg by body weight or the cow to which 1,25-(OH)₂D₃ was intravenously administered at 1.0 µg/kg by body weight, whereas no significant difference among groups arose in the values of Ca/Cre and iP/Cre.

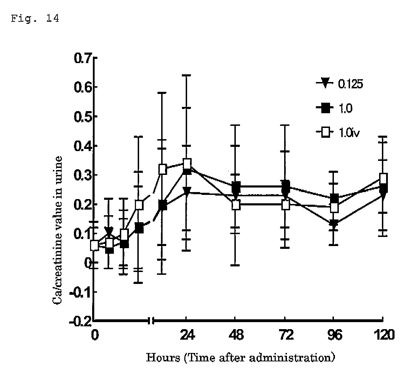

When 1,25-(OH)₂D₃ was administered at 1.0 µg/kg by body weight, the value of Ca/Cre in urine significantly rose via the vagina at 24 hours after administration and significantly rose via the vein at 12 hours and 24 hours after administration (Fig. 14).

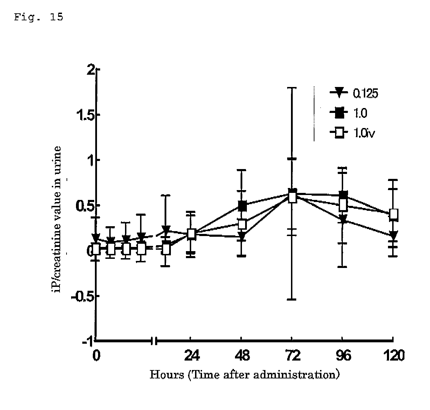

When 1,25-(OH)₂D₃ was administered at 1.0 µg/kg by body weight, the value of iP/Cre in urine significantly was significantly high via the vagina from 48 hours 120 hours after administration and via the vein from 72 hours to 120 hours after administration, as compared with the values immediately before administration (Fig. 15).

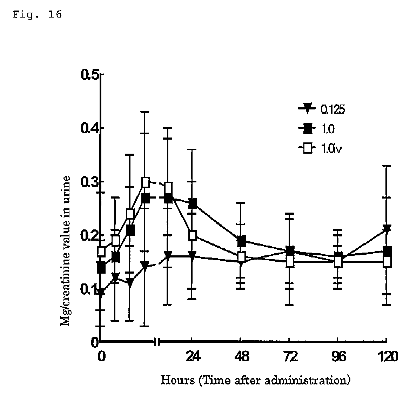

When 1,25-(OH)₂D₃ was administered at 1.0 µg/kg by body weight, the value of Mg/Cre in urine was significantly high via the vagina from 6 hours to 12 hours after administration and via the vein at 6 hours after administration (Fig. 16).

Individual bioavailability of the five cows was 71.1, 124.2, 113.3, 90.0, and 66.5%, respectively. It is noted that the bioavailability was determined by comparing an area under the plasma concentration-time curve (AUC) between the cow to which 1,25-(OH)₂D₃ was intravaginally administered at 1.0 µg/kg (by body weight) and the cow to which the same amount of 1,25-(OH)₂D₃ was intravenously administered, and a AUC ratio between intravaginal administration and intravenous administration was indicated by percentage.

Thepresent result indicates that 1,25-(OH)₂D₃ administered into the vaginal lumen of the ovariectomized cow is absorbed from the vaginal wall proportionately with a dose for all of the four different doses. In addition, the amount of 1,25-(OH)₂D₃ administered into the vaginal lumen has no dose-relationship with changes in calcium, inorganic phosphorus, and magnesium concentrations in plasma after administration. In spite of increases in calcium and inorganic phosphorus concentrations in plasma and a decrease in a magnesium concentration in plasma, the excretion of minerals to urine was not affected when 1, 25-(OH)₂D₃ was administered at 0.125 µg/kg by body weight. In this case, approximately 93% of 1,25-(OH)₂D₃ administered to the vaginal lumen was considered to enter into the circulatory system throughout the body.

Although it is known that a proportional rise in the AUC (area under the plasma concentration-time curve) of calcitriol in serum with an increase in a dose was not observed in oral administration (report by Muindi et al., (2002); Pharmacokinetics of high-dose oral calcitriol: Results from a phase I trial of calcitriol and paclitaxel. Clin. Pharmacol. Ther. 72: 648-659), the present result indicates that 1,25-(OH)₂D₃ is dose-dependently absorbed from the vaginal wall, so that the superiority of the vaginal administration of 1,25-(OH)₂D₃ is clear in this regard.

It is also known that the excretion of calcium into urine increases in cows to which 1,25-(OH)₂D₃ is intravenously administered at four dose levels (30, 90, 270, and 600 µg) and this increase is not directly related to the dose of steroid in the vein (report by Hoffsis et al., (1979); The use of 1,25-dihydroxycholecalciferol in the prevention of parturient hypocalcemia in dairy cows., Bovine Practitioner 13: 88-95). However, in the present result, the amount of calcium excreted into urine had no significant increase in the cow to which 1,25-(OH)₂D₃ was intravaginally administered at 0.125 µg/kg by body weight, in spite of the increase in a calcium concentration in plasma. Results from changes in the excretion of inorganic phosphorus and magnesium into urine were similar to that of the excretion of calcium into urine. Those results indicate that, of four doses for the administration of 1,25-(OH)₂D₃ to the vaginal lumen, a dose of 0.125 µg/kg by body weight is appropriate. However, in the lowest dose level (0.125 µg/kg by body weight), calcium and inorganic phosphorus concentrations in plasma also increased and magnesium in plasma decreased in a similar manner as the other dose levels.

The bioavailability of 1,25-(OH)₂D₃ via the vagina has not been known so far. The bioavailability of 1,25-(OH)₂D₃ at 24 hours after administration of calcitriol at a dose of 60 ng/kg to a young patient dialyzed for a long period is known to be 62% via the mouth and 67% via the peritoneal cavity (report by Salusky et al., (1990); Pharmacokinetics of calcitriol in continuous ambulatory and cycling peritoneal dialysis patients. Am. J. Kidney Dis. 16: 126-32). This report suggests that a first-pass effect in an intestine and/or a liver and a dialysate system of the peritoneal cavity decrease the bioavailability. The bioavailability shown in the present result (approximately 93%) is obviously higher than that in the report. Accordingly, the present result indicates that an effective route via which 1,25-(OH)₂D₃ is administered to a dairy cow is the vaginal cavity.

The present result indicates that 1,25-(OH)₂D₃ administered to the vaginal lumen is dose-dependently absorbed to a cow, and also indicates that a suitable dose for the administration of 1,25-(OH)₂D₃ to the vaginal lumen is the lowest dose (0.125 µg/kg by body weight) although the administration of 1,25-(OH)₂D₃ to a cow at a lower dose may affect blood and urine components.

### Industrial Applicability

According to a method of transvaginally administering a vitamin D derivative to a domesticmammal of the present invention, the prevention, cure, and/or treatment of diseases such as astasia caused by hypocalcemia of a domestic mammal, especially a cow are readily performed for the following reasons: (1) the vitamin D derivative is readily administered without medical equipment; (2) the substance is efficiently absorbed from the vagina without undergoing first-pass metabolism in the liver; (3) its delivery type in administration can have a wide choice of options for a form including a gel, a tablet, a microsphere, and CIDR (controlled internal drug release: a kind of tampon system); and (4) the vitamin D derivative is quickly absorbed because of the use of the vagina composed of tissue in which blood supply is well developed.

## Claims

1. A method of preventing hypocalcemia, **characterized in that** a vitamin D derivative is transvaginally administered to a domestic mammal.

2. The method of preventing hypocalcemia as claimed in claim 1, in which the domestic mammal is a cow.

3. The method of preventing hypocalcemia as claimed in claim 1 or 2, including administering an intravaginal insert containing the vitamin D derivative to a vaginal cavity.

4. The method of preventing hypocalcemia as claimed in any one of claims 1 to 3, in which the vitamin D derivative administered intravaginally is absorbed in the vaginal cavity of the domestic mammal to increase a calcium concentration in the body, thereby preventing the disease.

5. The method of preventing hypocalcemia as claimed in any one of claims 1 to 4, in which the vitamin D derivative is 1α-hydroxyvitamin D₃ or 1,25-dihydroxyvitamin D₃.

6. A method of curing hypocalcemia, including transvaginally administering a vitamin D derivative to a domestic mammal.

7. The method of curing hypocalcemia as claimed in claim 6, in which the domestic mammal is a cow.

8. The method of curing hypocalcemia as claimed in claim 6 or 7, including administering an intravaginal insert containing the vitamin D derivative to a vaginal cavity.

9. The method of curing hypocalcemia as claimed in any one of claims 6 to 8, in which the vitamin D derivative administered intravaginally is absorbed in the vaginal cavity of the domestic mammal to increase a calcium concentration in the body, thereby curing the disease.

10. The method of curing hypocalcemia as claimed in any one of claims 6 to 9, in which the vitamin D derivative is 1α-hydroxyvitamin D₃ or 1,25-dihydroxyvitamin D₃.

11. A method of treating hypocalcemia, **characterized in that** a vitamin D derivative is transvaginally administered to a domestic mammal.

12. The method of treating hypocalcemia as claimed in claim 11, in which the domestic mammal is a cow.

13. The method of treating hypocalcemia as claimed in claim 11 or 12, including administering an intravaginal insert containing the vitamin D derivative to a vaginal cavity.

14. The method of treating hypocalcemia as claimed in any one of claims 11 to 13, in which the vitamin D derivative administered intravaginally is absorbed in the vaginal cavity of the domestic mammal to increase a calcium concentration in the body, thereby treating the disease.

15. The method of treating hypocalcemia as claimed in any one of claims 11 to 14, in which the vitamin D derivative is 1α-hydroxyvitamin D₃ or 1,25-dihydroxyvitamin D₃.
